# EUROPÄISCHE PATENTSCHRIFT

(11) **EP 2 760 352 B1**
(45) Veröffentlichungstag und Bekanntmachung des Hinweises auf die Patenterteilung: **08.07.2015**
(21) Anmeldenummer: 12779039.2
(22) Anmeldetag: 24.10.2012
(51) Int. Cl.: A61B 17/32, B06B 3/00

(54) **SONOTRODE**
SONOTRODE
SONOTRODE

(30) Priorität: 18.01.2012 DE 102012200666
(43) Veröffentlichungstag der Anmeldung: 06.08.2014
(73) Patentinhaber: Söring GmbH, 25451 Quickborn (DE)
(72) Erfinder: RAD, Abtin Jamshidi, 22115 Hamburg (DE)
(74) Vertreter: Glawe, Delfs, Moll
(86) Internationale Anmeldenummer: PCT/EP2012/071053
(87) Internationale Veröffentlichungsnummer: WO 2013/107532

(56) Entgegenhaltungen:
- WO-A1-2010/049684
- CN-A- 101 789 713
- US-A1- 2001 011 176
- US-A1- 2010 121 197

## Beschreibung

Die Erfindung betrifft eine Sonotrode für ein ultraschallchirurgisches Instrument mit einem Schaft und einem Instrumentenkopf am distalen Ende des Schafts. Der Instrumentenkopf ist mit einer Schneidestruktur zum Bearbeiten von Knochen ausgerüstet. An dem Schaft ist eine helixförmige Ausnehmung ausgebildet. Die Erfindung betrifft außerdem ein Verfahren zum Herstellen einer solchen Sonotrode.

Solche Sonotroden können beispielsweise in der Zahnmedizin dazu verwendet werden, Knochenmaterial des Patienten zu durchtrennen oder abzutragen. Mit einem Ultraschallwandler wird eine hochfrequente mechanische Schwingung erzeugt. Die Sonotrode ist an den Ultraschallwandler angeschlossen und wird durch den Ultraschallwandler in Schwingung versetzt. Wenn die Schneidestruktur der Sonotrode mit dem Knochen in Kontakt gebracht wird, wird durch die hochfrequente Schwingung Knochenmaterial abgetragen.

Es ist bekannt, dass eine von dem Ultraschallwandler erzeugte Längsschwingung teilweise in eine Drehschwingung des Instrumentenkopfs umgesetzt werden kann, indem der Schaft mit helixförmigen Ausnehmungen versehen wird, US 2009/0236938 A1, US 2006/0041220 A1, WO 2010/049684 A1. Durch die helixförmigen Ausnehmungen verwindet sich der Schaft in sich, wenn die Längsschwingung des Ultraschallwandlers auf das proximale Ende des Schafts wirkt. Dadurch wird der Instrumentenkopf am distalen Ende des Schafts zu einer Drehschwingung angeregt.

Im Allgemeinen wird die Amplitude der Drehschwingung desto größer, je tiefer und je breiter die helixförmigen Ausnehmungen in das Material des Schafts eingreifen. Allerdings ist es nicht möglich, die Amplitude der Drehschwingung auf diese Weise beliebig zu vergrößern, weil der Schaft durch die Ausnehmungen nicht zu sehr geschwächt werden darf.

US 2010/0121197 A1 offenbart eine Sonotrode gemäß dem Oberbegriff des Anspruchs 1.

Der Erfindung liegt die Aufgabe zu Grunde, eine Sonotrode sowie ein zugehöriges Herstellungsverfahren vorzustellen, so dass eine wirksame Bearbeitung des Knochens erreicht wird. Ausgehend vom eingangs genannten Stand der Technik wird die Aufgabe gelöst mit den Merkmalen der unabhängigen Ansprüche. Vorteilhafte Ausführungsformen finden sich in den Unteransprüchen.

Erfindungsgemäß ist die helixförmige Ausnehmung des Schafts mit einem quer verlaufenden Absatz versehen.

Zunächst werden einige Begriffe erläutert. Ein Absatz in der helixförmigen Ausnehmung kann erzeugt werden, indem in einem Bereich der helixförmigen Ausnehmung tiefer in das Material des Schafts eingegriffen wird als in einem benachbarten Bereich der helixförmigen Ausnehmung. Der Übergang zwischen den beiden Bereichen wird als Absatz bezeichnet.

Die helixförmige Ausnehmung hat eine Längsausdehnung, die sich um den Schaft herumwindet. Der erfindungsgemäße Absatz ist quer zu der Längsausdehnung ausgerichtet, schließt also mit der Längsausdehnung einen Winkel ein. Der Absatz kann sich über die gesamte Breite der helixförmigen Ausnehmung erstrecken. Denkbar ist es auch, dass der Absatz sich nur über einen Teil der Breite der helixförmigen Ausnehmung erstreckt.

Die Erfindung hat erkannt, dass durch einen quer verlaufenden Absatz in der helixförmigen Ausnehmung die Amplitude der Drehschwingung am distalen Ende des Schafts verstärkt wird. Der Instrumentenkopf am distalen Ende des Schafts wird also in eine intensive Drehschwingung versetzt, was ein wirksames Bearbeiten des Knochenmaterials ermöglicht. Ihren Grund hat die vergrößerte Amplitude vermutlich darin, dass der Absatz eine Art Singularität für die Kraftübertragung innerhalb des Schafts bildet. Es kommt zu einem Bruch in der Kraftübertragung, der im Ergebnis eine verstärkte Drehschwingung am distalen Ende des Schafts zur Folge hat.

Der Absatz kann mit der Längsausdehnung der helixförmigen Ausnehmung einen Winkel von 90° einschließen. Als besonders wirksam hat es sich erwiesen, wenn der Winkel von 90° abweicht. Beispielsweise kann der Winkel zwischen dem Absatz und der Längsausdehnung der helixförmigen zwischen 10° und 80°, vorzugsweise zwischen 30° und 60° liegen.

Der Absatz kann eine Kante aufweisen, die quer zu der helixförmigen Ausnehmung verläuft. Die Kante kann sich geradlinig über die gesamte Breite der helixförmigen Ausnehmung erstrecken. Versuche haben aber gezeigt, dass es für die erfindungsgemäße Wirkung von Vorteil ist, wenn die Kante sich nur über einen Abschnitt der Breite der helixförmigen Ausnehmung geradlinig erstreckt und dann unterbrochen ist. Unterbrechung der Kante bedeutet in diesem Zusammenhang, dass die Kante sich nicht geradlinig in der gleichen Richtung fortsetzt. Indem die Kante selbst wieder unterbrochen ist, gibt es einen weiteren Bruch in der Kraftübertragung innerhalb des Schafts, was sich positiv auf die Amplitude der Schwingung am distalen Ende des Schafts auswirkt.

Die Amplitude der Schwingung am distalen Ende des Schafts kann weiter vergrößert werden, wenn die helixförmige Ausnehmung eine Mehrzahl von Absätzen mit den erwähnten Merkmalen aufweist. Die Absätze können sich parallel zueinander erstrecken. Mit der Längsachse der Sonotrode können die Absätze einen Winkel von 90° einschließen.

Die helixförmige Ausnehmung kann aus einer Mehrzahl von Ausfräsungen zusammengesetzt sein, wobei die Absätze am Übergang von einer Ausfräsung zur nächsten Ausfräsung gebildet sind. Eine helixförmige Ausnehmung kann beispielsweise zwischen acht und zwölf Ausfräsungen umfassen. Die Ausfräsungen können quer zur Längsausdehnung der helixförmigen Ausnehmungen ausgerichtet sein.

Erzeugt werden können die Ausfräsungen beispielsweise, indem ein Fräser zunächst in den Schaft einsticht und dann zur Seite (also rechtwinklig zur Achse des Fräsers und rechtwinklig zur Achse der Sonotrode) wieder aus dem Material des Schafts herausgeführt wird. Jede Ausfräsung hat dann an einem Ende eine Wand mit einer abgerundeten Kontur, während sie am anderen Ende direkt in die Umfangsfläche des Schafts übergeht. Um die Kante zu unterbrechen, kann der Fräser zunächst ein Stück parallel zur Längsachse bewegt werden, bevor die Bewegung quer zur Längsachse beginnt. Eine identische Ausfräsung lässt sich selbstverständlich durch einen umgekehrten Bewegungsablauf des Fräsers erreichen. Damit insgesamt eine helixförmige Ausnehmung entsteht, kann der Schaft zwischen der einen Ausfräsung und der nächsten Ausfräsung parallel zu seiner Längsachse verschoben werden und um die Längsachse gedreht werden, während der Fräser seine Ausgangsposition beibehält.

Die Sonotrode kann eine Mehrzahl von helixförmigen Ausnehmungen mit den beschriebenen Merkmalen aufweisen. Die Mehrzahl von helixförmigen Ausnehmungen ist vorzugsweise in demselben Längsabschnitt des Schafts angeordnet. Die helixförmigen Ausnehmungen können so miteinander verschränkt sein, dass sie gemeinsam eine helixförmige Struktur bilden. Die helixförmigen Ausnehmungen können sich beispielsweise über eine Länge zwischen 8 mm und 15 mm des Schafts erstrecken. In Umfangsrichtung kann sich die helixförmige Ausnehmung beispielsweise über einen Winkel von 180° erstrecken. Dabei kann der Durchmesser des Schafts beispielsweise zwischen 5 mm und 8 mm liegen. Der Winkel zwischen der Längsausdehnung der helixförmigen Ausnehmung und der Längsachse der Sonotrode kann zwischen 30° und 60° liegen.

Die Sonotrode kann vom proximalen Ende bis zum distalen Ende eine Länge zwischen beispielsweise 6 cm und 18 cm haben. Die Sonotrode kann so gestaltet sein, dass sie sich geradlinig vom proximalen Ende zum distalen Ende erstreckt.

Die Sonotrode ist vorzugsweise so auf den Ultraschallwandler des zugehörigen ultraschallchirurgischen Instruments abgestimmt, dass sich im zentralen Bereich der Sonotrode ein Schwingungsknoten bildet, wenn das Instrument mit der Sonotrode in Betrieb ist. Die helixförmige Ausnehmung ist vorzugsweise zwischen dem Schwingungsknoten und dem proximalen Ende der Sonotrode angeordnet. Dies bedeutet, dass die helixförmige Ausnehmung vom proximalen Ende ausgesehen vorzugsweise in der ersten Hälfte, weiter vorzugsweise im ersten Drittel der Sonotrode angeordnet ist.

Das Problem, dass der Schaft geschwächt wird, wenn die helixförmigen Ausnehmungen zu tief in das Material des Schafts eingreifen, stellt sich insbesondere dann, wenn sich im Inneren ein Kanal erstreckt, der beispielsweise dazu dienen kann, eine Spülflüssigkeit zum distalen Ende der Sonotrode zuzuführen. Die Vorzüge der Erfindung können folglich besonders zur Geltung kommen, wenn sich im Inneren des Schafts ein parallel zur Längsachse der Sonotrode ausgerichteter Kanal erstreckt. Der Kanal kann beispielsweise einen Durchmesser zwischen 0,5 mm und 1,5 mm, vorzugsweise zwischen 0,8 mm und 1,2 mm haben.

Die helixförmige Ausnehmung hat die Wirkung, dass eine Kraft, die am proximalen Ende der Sonotrode in Längsrichtung wirkt, teilweise in eine Drehbewegung umgesetzt wird. Die Schwingung des Instrumentenkopfs am distalen Ende der Sonotrode ist folglich eine Überlagerung aus einer Schwingung in Längsrichtung und einer Drehschwingung. Für die wirksame Bearbeitung des Knochens ist es von Vorteil, wenn nicht nur die Drehschwingung, sondern auch die Längsschwingung eine große Amplitude hat. Dies kann erreicht werden, indem die Sonotrode im Bereich zwischen der helixförmigen Ausnehmung und dem Instrumentenkopf mit einer Querschnittsverjüngung versehen wird.

Die Bearbeitung von Knochenmaterial kann weiter verbessert werden, wenn in dem Instrumentenkopf außer der Längsschwingung und der Drehschwingung noch weitere Bewegungsrichtungen überlagert sind. Dies kann dadurch erreicht werden, dass der Instrumentenkopf eine asymmetrische Gestalt bezogen auf die Längsachse der Sonotrode hat. Vom proximalen Ende aus betrachtet bildet der Instrumentenkopf dann eine Art Unwucht, was dazu führt, dass der Instrumentenkopf auch eine Bewegung in radialer Richtung vollführt.

Um Hitzeschäden am umliegenden Gewebe zu vermeiden, ist es wünschenswert, dass die vom Knochen abgetragenen Späne zügig aus dem Operationsfeld entfernt werden. Würden die Späne im Operationsfeld bleiben, würden sie als eine Art Wärmespeicher wirken und die Hitzeeinwirkung auf das umliegende Gewebe verstärken. Für den Abtransport der Späne ist es förderlich, wenn der Instrumentenkopf in sich eine Nickbewegung vollführt. Der Instrumentenkopf wirkt dann nach Art einer Schaufel, die die Späne abtransportiert. Zu einer solchen Nickbewegung kann der Instrumentenkopf veranlasst werden, indem die distale Stirnfläche der Sonotrode mit einer Sackbohrung versehen wird. Der Durchmesser der Sackbohrung kann beispielsweise 0,2 mm bis 0,3 mm betragen. Die Länge der Sackbohrung kann beispielsweise zwischen 1,2 mm und 1,8 mm liegen. Durch die Sackbohrung wird die Struktur des Instrumentenkopfs gezielt geschwächt, so dass der Instrumentenkopf die gewünschte In-Sich-Beweglichkeit erhält. Das Merkmal der Sackbohrung hat eigenständigen erfinderischen Gehalt, auch ohne dass der Schaft mit einer helixförmigen Ausnehmung versehen ist.

Die Sackbohrung kann sich parallel zur Längsachse der Sonotrode erstrecken und einen Abstand zur zentralen Achse der Sonotrode haben. Vorzugsweise ist die Sackbohrung im Bereich zwischen der zentralen Achse und der Schneidestruktur angeordnet. In einer vorteilhaften Ausführungsform ist die Stirnfläche mit zwei solchen Sackbohrungen versehen. Solche Sackbohrungen haben außerdem die Wirkung, dass das Schwingungsprofil dahingehend optimiert wird, dass die Rückkopplung von Kräften vom Instrumentenkopf auf den Ultraschallwandler verringert wird und so eine Überlastung des Ultraschallwandlers verhindert wird.

Die Erfindung betrifft auch ein chirurgisches Instrument mit einem Ultraschallwandler und einer an den Ultraschallwandler angeschlossenen erfindungsgemäßen Sonotrode. Das Instrument kann mit einer Zuleitung versehen sein, über die dem Kanal im Inneren des Schafts eine Flüssigkeit zugeführt werden kann. Zusätzlich oder alternativ dazu kann die Zuleitung so gestaltet sein, dass eine Flüssigkeit außen an der Sonotrode entlang geleitet wird.

Die Erfindung betrifft außerdem ein Verfahren zum Herstellen einer solchen Sonotrode. Bei dem Verfahren wird ein Sonotroden-Zwischenprodukt bereitgestellt, das einen Schaft und einen Instrumentenkopf am distalen Ende des Schafts umfasst. Es wird eine Mehrzahl von Ausfräsungen in dem Schaft erzeugt. Jeweils zwei Ausfräsungen sind derart zueinander benachbart, dass am Übergang der Ausfräsungen ein Absatz gebildet wird. Die benachbarten Ausnehmungen sind jeweils sowohl in Längsrichtung der Sonotrode als auch in Umfangsrichtung relativ zueinander versetzt, so dass die Ausfräsungen in ihrer Summe eine helixförmige Ausnehmung bilden.

Die Ausfräsungen können sich parallel zueinander erstrecken, wobei jeweils die größte Ausdehnung der Ausfräsung einen rechten Winkel mit der Längsachse der Sonotrode einschließt. In einer vorteilhaften Ausführungsform sticht der Fräser zunächst in radialer Richtung in den Schaft ein und wird dann in seitlicher Richtung aus dem Material des Schafts herausgeführt. Der Bewegungsablauf des Fräsers kann auch umgekehrt sein. Der Fräser kann beispielsweise einen Durchmesser zwischen 0,5 mm und 1,5 mm, vorzugsweise zwischen 0,8 mm und 1,2 mm haben. Die größte Tiefe der Ausnehmung, also die größte Strecke, die der Fräser von der Oberfläche des Schafts in das Material eindringt, kann beispielsweise zwischen 0,5 mm und 1,5 mm, vorzugsweise zwischen 0,8 mm und 1,2 mm liegen.

Das Verfahren kann mit weiteren Merkmalen fortgebildet werden, die oben mit Bezug auf die erfindungsgemäße Sonotrode beschrieben wurden. Die Erfindung betrifft außerdem eine Sonotrode, die durch das erfindungsgemäße Verfahren erhältlich ist.

Die Erfindung wird nachfolgend unter Bezugnahme auf die beigefügten Zeichnungen anhand einer vorteilhaften Ausführungsform beispielhaft beschrieben. Es zeigen:
- Fig. 1:: eine Seitenansicht eines erfindungsgemäßen ultraschallchirurgischen Instruments;
- Fig. 2:: eine Seitenansicht einer erfindungsgemäßen Sonotrode;
- Fig. 3:: einen Ausschnitt aus Fig. 2 in vergrößerter Darstellung; und
- Fig. 4:: ein Detail aus Fig. 2 in vergrößerter Darstellung und aus einer anderen Perspektive.

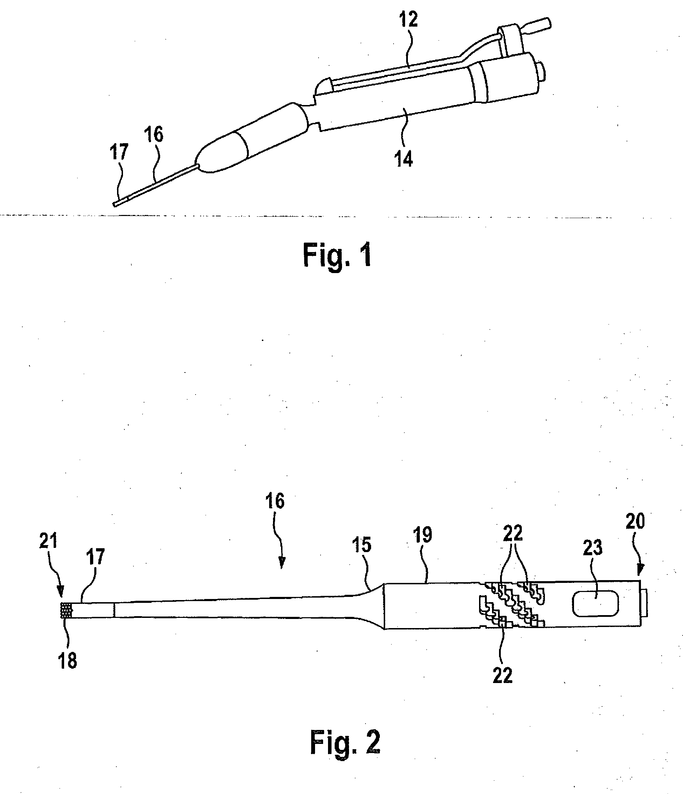

Ein ultraschallchirurgisches Instrument in Fig. 1 umfasst an seinem hinteren Ende einen Handgriff 14, über den der Chirurg das Instrument führen kann. Im Inneren des Instruments ist ein in Fig. 1 nicht sichtbarer Ultraschallwandler angeordnet, der als Eingangssignal ein elektrisches Wechselspannungssignal von einem in Fig. 1 ebenfalls nicht dargestellten Signalgenerator erhält. Die Frequenz der Wechselspannung kann beispielsweise zwischen 20 kHz und 40 kHz liegen. Der Ultraschallwandler umfasst ein Piezoelement, mit dem das elektrische Signal in eine mechanische Schwingung umgewandelt wird, die in Längsrichtung des Instruments ausgerichtet ist. Die mechanische Schwingung wird auf eine Sonotrode 16 übertragen, die gemäß Fig. 2 einen Schaft 19 mit einem Instrumentenkopf 17 am distalen Ende umfasst. Die in Fig. 1 unten und in Fig. 2 in der Bildebene dargestellte Fläche des Instrumentenkopfs 17 ist mit einer Schneidestruktur 18 ausgerüstet und dient als Schneidfläche. Wird der in Schwingung stehende Instrumentenkopf 17 mit der in Schwingung stehenden Schneidestruktur 18 an einen Knochen herangeführt, wird Knochenmaterial abgetragen.

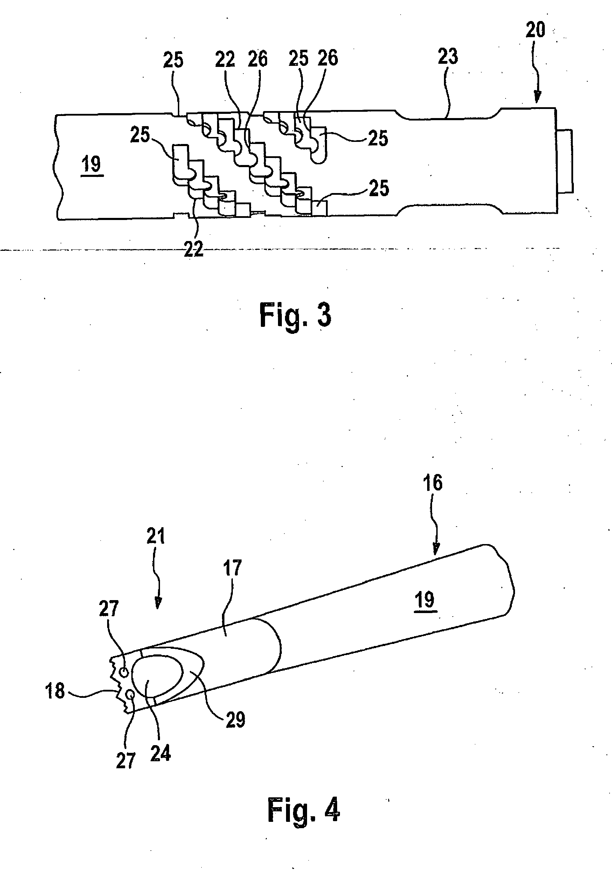

Das ultraschallchirurgische Instrument umfasst außerdem eine in Fig. 1 sichtbare Leitung 12, um dem Operationsfeld eine Spülflüssigkeit zuführen zu können. Die Spülflüssigkeit wird durch einen im Inneren der Sonotrode 16 angeordneten Kanal 24 zum Instrumentenkopf 17 geleitet und kann dort in das Operationsfeld austreten. Das distale Ende des Kanals 24 ist in Fig. 4 sichtbar. Wahlweise kann die Spülflüssigkeit auch außen an der Sonotrode entlang geleitet werden.

Die Sonotrode 16 erstreckt sich gemäß Fig. 2 von einem proximalen Ende 20, über das die Sonotrode 16 mit dem Handstück des chirurgischen Instruments verbunden werden kann, geradlinig bis zu einem distalen Ende 21, an dem der Instrumentenkopf 17 angeordnet ist. Der Schaft 19 geht mit einer Querschnittsverjüngung 15 von einem dickeren Bereich nahe dem proximalen Ende 20 in einen schlankeren Bereich nahe dem distalen Ende 21 über. Im dickeren Bereich hat der Schaft einen Durchmesser von 6,5 mm. Die Länge der Sonotrode vom proximalen Ende 20 bis zum distalen Ende 21 beträgt etwa 10 cm. Am proximalen Ende 20 ist der Schaft 19 mit Vertiefungen 23 versehen, an denen mit einem Schraubenschlüssel angegriffen werden kann, um die Sonotrode 16 mit dem Handstück zu verschrauben bzw. von dem Handstück zu lösen.

Wenn die Sonotrode 16 in Betrieb ist und durch den Ultraschallwandler in Schwingung versetzt wird, bildet sich etwa mittig zwischen dem proximalen Ende 20 und dem distalen Ende 21 ein Schwingungsknoten. Im Bereich zwischen dem Schwingungsknoten und dem proximalen Ende 20 ist der Schaft 19 mit einer helixförmigen Struktur versehen, die aus vier miteinander verschränkten helixförmigen Ausnehmungen 22 besteht. Von der helixförmigen Struktur, die in Fig. 3 vergrößert dargestellt ist, ist eine helixförmige Ausnehmung 22 im Wesentlichen vollständig sichtbar und sind zwei helixförmige Ausnehmungen 22 teilweise sichtbar, während die vierte Ausnehmung durch den Schaft 19 verdeckt und deswegen nicht sichtbar ist.

Die helixförmigen Ausnehmungen 22 haben eine Längsausdehnung, mit der sie sich um den Schaft 19 herum winden. In ihrer Längsausdehnung schließen die helixförmigen Ausnehmungen 22 mit der Längsachse des Schafts 19 einen Winkel von 45° ein. Die helixförmigen Ausnehmungen 22 haben eine Längsausdehnung von etwa 1,5 cm. Die Breite rechtwinklig zur Längsausdehnung, die sich nicht eindeutig bestimmen lässt, da die helixförmigen Ausnehmungen 22 keinen geradlinigen Rand haben, liegt in der Größenordnung von 2 mm.

Jede der helixförmigen Ausnehmungen 22 setzt sich aus zehn Ausfräsungen 25 zusammen, deren größte Ausdehnung jeweils rechtwinklig zur Längsachse des Schafts 19 ausgerichtet ist. Die Ausfräsungen 25 sind jeweils sowohl in Längsrichtung als auch in Umfangsrichtung des Schafts 19 relativ zueinander versetzt, so dass in Summe die Helixform entsteht.

Die jeweils erste Ausfräsung 25 einer helixförmigen Ausnehmung 22 (in Fig. 3 jeweils die rechte Ausnehmung 22) wird erzeugt, indem mit einem Fräser senkrecht in das Material des Schafts 19 eingestochen wird und der Fräser dann zur Seite (in Fig. 3 nach oben) geführt wird, bis er aus dem Material des Schafts 19 austritt.

Um die nächste Ausfräsung 25 zu erzeugen, kann der Fräser wieder in seine Ausgangsposition zurückgefahren werden. Der Schaft 19 wird in Längsrichtung relativ zu dem Fräser verschoben und etwas um seine Längsachse gedreht, so dass der Fräser in einer neuen Position in das Material des Schafts 19 einstechen kann, um die nächste Ausfräsung 25 zu erzeugen. Die Verschiebung in Längsrichtung beträgt etwa die Hälfte der Breite der Ausfräsung 25, so dass eine Überschneidung mit der ersten Ausfräsung 25 besteht, wenn der Fräser für die zweite Ausfräsung 25 in das Material einsticht. Der Fräser wird dann zunächst ein Stück parallel zur Längsachse des Schafts 19 geführt, bevor er mit der Bewegung in seitlicher Richtung aus dem Material herausgeführt wird.

Da der Fräser bei der zweiten Ausnehmung 22 aus einer anderen Winkelposition in den Schaft 19 einsticht als bei der ersten Ausfräsung 25, entsteht zwischen den Ausfräsungen 25 eine Kante 26. Indem der Fräser zunächst in Längsrichtung geführt wird, wird die Kante 26 zwischen der ersten Ausfräsung 25 und der zweiten Ausfräsung 25 unterbrochen, die anderenfalls geradlinig wäre. In Fig. 3 zeigt sich die Unterbrechung der Kante 26 jeweils als halbkreisförmige Vertiefung in den Ausnehmungen 25.

Durch zehn auf diese Weise zueinander benachbarte Ausfräsungen 25 wird eine helixförmige Ausnehmung 22 erzeugt. Die Kanten 26 bilden Absätze im Sinne der Erfindung, die quer zu der helixförmigen Ausnehmung 22 verlaufen. Der geschilderte Ablauf beim Erzeugen der helixförmigen Ausnehmung 22 dient lediglich der Veranschaulichung. Es sind auch andere Abläufe möglich, etwa in dem die Ausfräsungen 25 in anderer Reihenfolge hergestellt werden oder der Fräser sich in entgegengesetzter Richtung bewegt.

Durch die helixförmigen Ausnehmungen 22 wird die Längsschwingung, in die das proximale Ende 20 der Sonotrode 16 durch den Ultraschallwandler versetzt wird, zum Teil in eine Drehschwingung umgesetzt. Der Instrumentenkopf 17 am distalen Ende wird dadurch in eine komplexe Bewegung versetzt, die Drehschwingungen und Längsschwingungen umfasst. Durch die Kanten 26, die die helixförmigen Ausnehmungen 22 quer unterbrechen, hat die Drehschwingung des Instrumentenkopfs 17 eine größere Amplitude als bei klassischen helixförmigen Ausnehmungen 22. Durch die Querschnittsverjüngung 15 wird die Längsamplitude verstärkt.

In Fig. 4 ist das distale Ende 21 der Sonotrode 16 vergrößert dargestellt, wobei die Perspektive so gewählt ist, dass die Stirnfläche mit dem Austritt des Kanals 24 sichtbar ist, während die Schneidestruktur 18 schräg nach hinten weist und verdeckt ist. Die distale Stirnfläche der Sonotrode 16 ist mit zwei Sackbohrungen 27 versehen, die sich parallel zu dem Kanal 24 erstrecken und zwischen dem Kanal 24 und der Schneidestruktur 18 angeordnet sind. Die Sackbohrungen haben einen Durchmesser zwischen 0,2 mm und 0,3 mm und eine Länge von etwa 1,5 mm. Durch die Sackbohrungen 27 und eine Abschrägung 29 erhält der Instrumentenkopf 17 eine asymmetrische Gestalt bezogen auf die Längsachse der Sonotrode 16. Diese asymmetrische Gestalt führt dazu, dass der Instrumentenkopf 17 in sich eine Nickbewegung vollführt. Durch die Nickbewegung werden Späne, die mit der Schneidestruktur 18 vom Knochen abgetragen werden, besonders wirksam abtransportiert.

Mit der Erfindung wird also eine Sonotrode vorgestellt, bei der der Instrumentenkopf 17 eine mehrdimensionale Schwingung vollführt, durch die Knochenmaterial mit hoher Wirksamkeit abgetragen werden kann. Durch die zusätzliche Nickbewegung des Instrumentenkopfs 17 werden von dem Knochenmaterial gelöste Späne gut abtransportiert.

## Patentansprüche

1. Sonotrode für ein ultraschallchirurgisches Instrument mit einem Schaft (19) und einem Instrumentenkopf (17) an einem distalen Ende (21) des Schafts (19), wobei der Instrumentenkopf (17) mit einer Schneidestruktur (18) zum Bearbeiten von Knochen ausgerüstet ist und wobei der Schaft (19) eine helixförmige Ausnehmung (22) aufweist, **dadurch gekennzeichnet, dass** die helixförmige Ausnehmung (22) mit einem quer verlaufenden Absatz (26) versehen ist.

2. Sonotrode nach Anspruch 1, **dadurch gekennzeichnet, dass** der Absatz (26) mit der Längsausdehnung der helixförmigen Ausnehmung einen Winkel zwischen 10° und 80°, vorzugsweise zwischen 30° und 60° einschließt.

3. Sonotrode nach Anspruch 1 oder 2, **dadurch gekennzeichnet, dass** der Absatz eine Kante (26) aufweist, die quer zu der helixförmigen Ausnehmung (22) verläuft.

4. Sonotrode nach Anspruch 3, **dadurch gekennzeichnet, dass** die Kante (26) sich geradlinig über einen Abschnitt der helixförmigen Ausnehmung (22) erstreckt und dann unterbrochen ist.

5. Sonotrode nach einem der Ansprüche 1 bis 4, **dadurch gekennzeichnet, dass** die helixförmige Ausnehmung (22) eine Mehrzahl von Absätzen (26) aufweist.

6. Sonotrode nach einem der Ansprüche 1 bis 5, **dadurch gekennzeichnet, dass** die helixförmige Ausnehmung (22) aus einer Mehrzahl von Ausfräsungen (22) zusammengesetzt ist.

7. Sonotrode nach einem der Ansprüche 1 bis 6, **dadurch gekennzeichnet, dass** der Schaft (19) eine Mehrzahl von helixförmigen Ausnehmungen (22) aufweist.

8. Sonotrode nach einem der Ansprüche 1 bis 7, **dadurch gekennzeichnet, dass** sich im Inneren des Schafts (19) ein Kanal (24) erstreckt, über den eine Flüssigkeit zum distalen Ende (21) der Sonotrode (16) zugeführt werden kann.

9. Sonotrode nach einem der Ansprüche 1 bis 8, **dadurch gekennzeichnet, dass** zwischen der helixförmigen Ausnehmung (22) und dem Instrumentenkopf (17) eine Querschnittsverjüngung (15) vorgesehen ist.

10. Sonotrode nach einem der Ansprüche 1 bis 9, **dadurch gekennzeichnet, dass** der Instrumentenkopf (17) bezogen auf die Längsachse der Sonotrode (16) eine asymmetrische Gestalt hat.

11. Sonotrode nach einem der Ansprüche 1 bis 10, **dadurch gekennzeichnet, dass** die distale Stirnfläche der Sonotrode (16) mit einer Sackbohrung (27) versehen ist.

12. Chirurgisches Instrument mit einem Ultraschallwandler und einer an den Ultraschallwandler angeschlossenen Sonotrode (16), **dadurch gekennzeichnet, dass** die Sonotrode (16) nach einem der Ansprüche 1 bis 11 ausgebildet ist.

13. Verfahren zum Herstellen einer Sonotrode mit folgenden Schritten:
a. Bereitstellen eines Sonotroden-Zwischenprodukts, das einen Schaft (19) und einen Instrumentenkopf (17) am distalen Ende (21) des Schafts (19) umfasst.
b. Erzeugen einer Mehrzahl von Ausfräsungen (25) in dem Schaft (19), wobei jeweils zwei benachbarte Ausfräsungen (25) derart angeordnet sind, dass am Übergang der Ausfräsungen (25) ein Absatz (26) gebildet wird, und wobei jeweils benachbarte Ausfräsungen (25) sowohl in Längsrichtung der Sonotrode (16) als auch in Umfangsrichtung relativ zueinander versetzt sind, so dass die Ausfräsungen (25) in ihrer Summe eine helixförmige Ausnehmung (22) bilden.

## Claims

1. Sonotrode for an ultrasonic surgical instrument having a shaft (19) and an instrument head (17) at a distal end (21) of the shaft (19), the instrument head (17) being equipped with a cutting structure (18) for the treatment of bones and the shaft (19) having a helical cut-out (22), **characterized in that** the helical cut-out (22) is provided with a transversely running shoulder (26).

2. Sonotrode according to Claim 1, **characterized in that** the shoulder (26) includes an angle of between 10° and 80°, preferably of between 30° and 60°, with the longitudinal extent of the helical cut-out.

3. Sonotrode according to Claim 1 or 2, **characterized in that** the shoulder has an edge (26) which runs transversely to the helical cut-out (22).

4. Sonotrode according to Claim 3, **characterized in that** the edge (26) extends in a straight line over a section of the helical cut-out (22) and is then interrupted.

5. Sonotrode according to one of Claims 1 to 4, **characterized in that** the helical cut-out (22) has a plurality of shoulders (26).

6. Sonotrode according to one of Claims 1 to 5, **characterized in that** the helical cut-out (22) is composed of a plurality of milled-out portions (22).

7. Sonotrode according to one of Claims 1 to 6, **characterized in that** the shaft (19) has a plurality of helical cut-outs (22).

8. Sonotrode according to one of Claims 1 to 7, **characterized in that** extending within the interior of the shaft (19) there is a channel (24) by means of which a liquid can be supplied to the distal end (21) of the sonotrode (16).

9. Sonotrode according to one of Claims 1 to 8, **characterized in that** a cross-sectional taper (15) is provided between the helical cut-out (22) and the instrument head (17).

10. Sonotrode according to one of Claims 1 to 9, **characterized in that** the instrument head (17) has an asymmetric shape with respect to the longitudinal axis of the sonotrode (16).

11. Sonotrode according to one of Claims 1 to 10, **characterized in that** the distal end face of the sonotrode (16) is provided with a blind hole (27).

12. Surgical instrument with an ultrasound transducer and a sonotrode (16) connected to the ultrasound transducer, **characterized in that** the sonotrode (16) is embodied according to one of Claims 1 to 11.

13. Method for producing a sonotrode, comprising the following steps:
a. providing a sonotrode semi-finished product which comprises a shaft (19) and an instrument head (17) at the distal end (21) of the shaft (19);
b. creating a plurality of milled-out portions (25) in the shaft (19), with respectively two adjacent milled-out portions (25) being arranged such that a shoulder (26) is formed at the transition between the milled-out portions (25) and with respectively adjacent milled-out portions (25) being offset relative to one another both in the longitudinal direction of the sonotrode (16) and in the circumferential direction such that the milled-out portions (25) overall form a helical cut-out (22).

## Revendications

1. Sonotrode pour un instrument chirurgical à ultrasons avec une tige (19) et une tête d'instrument (17) à une extrémité distale (21) de la tige (19), la tête d'instrument (17) étant munie d'une structure de coupe (18) pour le traitement d'os et la tige (19) présentant un évidement hélicoïdal (22), **caractérisée en ce que** l'évidement hélicoïdal (22) est muni d'un épaulement (26) à extension transversale.

2. Sonotrode selon la revendication 1, **caractérisée en ce que** l'épaulement (26) forme, avec l'extension longitudinale de l'évidement hélicoïdal, un angle entre 10° et 80°, de préférence entre 30° et 60°.

3. Sonotrode selon la revendication 1 ou 2, **caractérisée en ce que** l'épaulement présente une arête (26) qui s'étend transversalement par rapport à l'évidement hélicoïdal (22).

4. Sonotrode selon la revendication 3, **caractérisée en ce que** l'arête (26) s'étend en ligne droite sur une portion de l'évidement hélicoïdal (22) puis est interrompue.

5. Sonotrode selon l'une des revendications 1 à 4, **caractérisée en ce que** l'évidement hélicoïdal (22) comprend une pluralité d'épaulements (26).

6. Sonotrode selon l'une des revendications 1 à 5, **caractérisée en ce que** l'évidement hélicoïdal (22) est constitué d'une pluralité de fraisages (22).

7. Sonotrode selon l'une des revendications 1 à 6, **caractérisée en ce que** la tige (19) comprend une pluralité d'évidements hélicoïdaux (22).

8. Sonotrode selon l'une des revendications 1 à 7, **caractérisée en ce que**, à l'intérieur de la tige (19), s'étend un canal (24) par l'intermédiaire duquel un liquide peut être introduit à l'extrémité distale (21) de la sonotrode (16).

9. Sonotrode selon l'une des revendications 1 à 8, **caractérisée en ce que**, entre l'évidement hélicoïdal (22) et la tête d'instrument (17), se trouve un rétrécissement de section transversale (15).

10. Sonotrode selon l'une des revendications 1 à 9, **caractérisée en ce que** la tête d'instrument (17) présente une forme asymétrique par rapport à l'axe longitudinal de la sonotrode (16).

11. Sonotrode selon l'une des revendications 1 à 10, **caractérisée en ce que** la face frontale distale de la sonotrode (16) est munie d'un alésage borgne (27).

12. Instrument chirurgical avec un convertisseur à ultrasons et une sonotrode (16) branchée au convertisseur à ultrasons, **caractérisé en ce que** la sonotrode (16) est conçue selon l'une des revendications 1 à 11.

13. Procédé de fabrication d'une sonotrode comprenant les étapes suivantes :
a. Préparation d'une ébauche intermédiaire de sonotrode, qui comprend une tige (19) et une tête d'instrument (17) sur l'extrémité distale (21) de la tige (19),
b. Réalisation d'une pluralité de fraisages (25) dans la tige (19), deux fraisages (25) adjacents étant disposés de façon à ce que, au niveau de la transition entre les fraisages (25), un épaulement (26) soit formé et des fraisages (25) adjacents soient décalés entre eux aussi bien dans la direction longitudinale de la sonotrode (16) que dans la direction circonférentielle, de façon à ce que les fraisages (25) forment ensemble un évidement hélicoïdal (22).
